# EUROPEAN PATENT SPECIFICATION

(11) **EP 3 318 237 B1**
(45) Date of publication and mention of the grant of the patent: **22.05.2019**
(21) Application number: 16817595.8
(22) Date of filing: 23.05.2016
(51) Int. Cl.: A61F 13/533, A61F 13/53, A61F 13/536, A61F 13/539, A61F 13/534

(54) **ABSORBENT ARTICLE**
SAUGFÄHIGER ARTIKEL
ARTICLE ABSORBANT

(30) Priority: 30.06.2015 JP 2015132113
(43) Date of publication of application: 09.05.2018
(73) Proprietor: Unicharm Corporation, Ehime 799-0111 (JP)
(72) Inventor: ONISHI, Kazuaki, Kanonji-shi Kagawa 769-1602 (JP)
(74) Representative: Staeger & Sperling Partnerschaftsgesellschaft mbB
(86) International application number: PCT/JP2016/065212
(87) International publication number: WO 2017/002486

(56) References cited:
- WO-A1-2011/071070
- WO-A1-2012/081282
- CN-A- 104 684 518
- JP-A- 2002 119 539
- JP-A- 2002 119 539
- JP-A- 2002 165 834
- JP-A- 2009 028 186

## Description

### Technical Field

The present invention relates to an absorbent article such as a disposable diaper.

### Background Art

In recent years, for absorbent articles such as disposable diapers, efforts have been made to form grooves running in the lengthwise direction and short direction in the absorbent body, for the purpose of efficiently absorbing excreted fluid such as urine, that has been discharged by the wearer, into the absorbent body and to impart flexibility to the absorbent article in order to improve the fitting property.

As an example of such an absorbent article, PTL 1 discloses a longitudinal absorbent article having a top sheet disposed on the skin contact surface side, a back sheet disposed on the non-skin contact surface side, and an absorbent body lying between both sheets, wherein the absorbent body has high-basis-weight sections with relatively high basis weight and low-basis-weight sections with relatively low basis weight, adjacent to the high-basis-weight sections, the low-basis-weight sections being maldistributed on the skin contact surface side in the thickness direction of the absorbent article, the absorbent article having grooves where the top sheet and absorbent body are recessed in an integral manner, running in the lengthwise direction of the absorbent article, the grooves being formed straddling the high-basis-weight sections and the low-basis-weight sections, the grooves including shallow groove sections that are relatively shallowly recessed and deep groove sections that are relatively deeply recessed, formed alternately along the lengthwise directions of the grooves, the shallow groove sections being longer in the lengthwise directions of the grooves than the deep groove sections. With the absorbent article disclosed in PTL 1, it is possible to rapidly absorb and diffuse excreted fluid such as urine that has been discharged from the wearer, and to eliminate discomfort caused by wetting of the wearer, while providing suitably flexibility and an excellent fitting property, despite the presence of the leak-resistant grooves.

### Citation List

### Patent Literature

[PTL 1] Japanese Unexamined Patent Publication No. 2012-245130

### Summary of Invention

### Technical Problem

With the absorbent article disclosed in PTL 1, however, despite the fact that the structure of the groove sections of the absorbent body is maintained before absorption of excreted fluid such as urine, and it therefore tends to fold on the groove sections as origins and thereby exhibits suitable flexibility and an excellent fitting property, in some cases, after having absorbed excreted fluid such as urine, and especially after having absorbed excreted fluid several times, the absorbent body may expand due to the absorbed excreted fluid making the widths of the groove sections narrower, often such that the groove sections essentially disappear, and therefore the groove sections become less able to serve as origins for folding and, as a result, sometimes the flexibility is reduced and the fitting property is impaired. Furthermore, with the absorbent article disclosed in PTL 1, the groove sections of the absorbent body are formed by embossing, and therefore excreted fluid such as urine has often been less able to permeate in the thickness direction of the absorbent body at the bottom sections of the groove sections. When excreted fluid such as urine is poorly able to permeate in the thickness direction of the absorbent body (i.e., the absorption rate of fluid is slow), it can potentially lead to rewetting or an inferior feel during wear.

It is therefore an object of the present invention to provide an absorbent article having an excellent fitting property and also an excellent rewetting property and feel during wear, not only before absorption but also after absorption of excreted fluid such as urine.

### Solution to Problem

The absorbent article according to one aspect (aspect 1) of the invention is an absorbent article having a lengthwise direction and a widthwise direction and including a liquid-permeable sheet, a liquid-impermeable sheet and an absorbent body between the liquid-permeable sheet and liquid-impermeable sheet, wherein the absorbent body comprises an absorbent core having a first surface on the side facing the liquid-permeable sheet, and a second surface on the side facing the liquid-impermeable sheet, and a core wrap covering the absorbent core, the absorbent core comprising a lengthwise groove section that is a groove section indented from the second surface and extending in the lengthwise direction, and with a bottom part, and having lower basis weight than the average basis weight of the absorbent core, the lengthwise groove section including a plurality of through-holes running from the first surface to the second surface, and the core wrap being joined to the bottom part of the lengthwise groove section.

Since in the absorbent article of the aspect 1, the absorbent core of the absorbent body comprises the lengthwise groove section with the specific structure described above and the lengthwise groove section has a plurality of through-holes, excreted fluid supplied to the absorbent body can rapidly permeate in the thickness direction of the absorbent core through the plurality of through-holes, and excreted fluid can be rapidly supplied to an excreted fluid diffusion channel that is formed by the lengthwise groove section, thus allowing an excellent absorption property (particularly absorption rate and diffusibility) and rewetting property to be obtained for the absorbent article. Excreted fluid that has reached the lengthwise groove section from the surface of the liquid-permeable sheet side of the absorbent core via the through-holes is diffused in the lengthwise direction along the lengthwise groove section, while being absorbed from the side walls of the lengthwise groove section, and therefore the excreted fluid can be absorbed through a wide region in the lengthwise direction of the absorbent body, and a more excellent absorption property (particularly absorption efficiency) and rewetting property can be obtained for the absorbent article.

In addition, since the absorbent body in the absorbent article of aspect 1 has a structure in which a core wrap is joined to the bottom part of the lengthwise groove section of the absorbent core, even when the absorbent core has absorbed excreted fluid and expanded, and the absorbent core swells in the widthwise direction at the pair of side walls of the lengthwise groove section and deforms in a manner that would plug the lengthwise groove section, since the core wrap joined to the bottom part of the lengthwise groove section lies between the pair of side walls that have swelled in the widthwise direction, it is possible to ensure a fixed space in the lengthwise groove section running in the lengthwise direction, and it is possible to maintain the function of the lengthwise groove section as a folding origin and the function as an excreted fluid diffusion channel. Consequently, the absorbent article of aspect 1 has flexibility and an excellent fitting property even after absorption of excreted fluid, and can maintain the aforementioned excellent absorption property and rewetting property.

An absorbent article according to another aspect (aspect 2) of the invention is the absorbent article of aspect 1 wherein the lengthwise groove section has a width of 0.5 to 3.0 times the thickness of the absorbent body.

Since the lengthwise groove section of the absorbent article of aspect 2 has a width of 0.5 to 3.0 times the thickness of the absorbent body, even when the absorbent core has absorbed excreted fluid and expanded, it does not expand to the point of mutual contact between the pair of side walls that have expanded in the widthwise direction and the lengthwise groove section is less likely to be plugged, and therefore the fixed space extending in the lengthwise direction can be more easily ensured in the lengthwise groove section. As a result, the effect of aspect 1 can be more reliably obtained.

The absorbent article according to another aspect (aspect 3) of the invention is the absorbent article of aspect 1 or 2 wherein the lengthwise groove section comprises a compressed section where a portion of the bottom part is compressed in the widthwise direction, the compressed section running continuously or intermittently along the lengthwise groove section.

Since the absorbent article of aspect 3 comprises the aforementioned compressed section in the lengthwise groove section, even when the absorbent core has absorbed excreted fluid and has expanded, expansion of the absorbent core is minimized by the compressed section at the bottom part of the lengthwise groove section and the lengthwise groove section is less likely to become plugged, thus further helping to ensure the fixed space running in the lengthwise direction in the lengthwise groove section.

The absorbent article according to yet another aspect (aspect 4) of the invention is the absorbent article of any of aspects 1 to 3, wherein the compressed section is formed by compressing the absorbent core and the core wrap.

Since the compressed section of the absorbent article of aspect 4 is formed by compressing the absorbent core and core wrap, even when the absorbent core has absorbed excreted fluid and has expanded, the core wrap that is joined to the compressed section at the bottom part of the lengthwise groove section more easily lies between the pair of side walls that have expanded in the widthwise direction, thus further helping to ensure the fixed space running in the lengthwise direction in the lengthwise groove section. As a result, the effects of the absorbent articles of aspects 1 to 3 can be more reliably exhibited.

The absorbent article according to yet another aspect (aspect 5) of the invention is the absorbent article of any of aspects 1 to 4 wherein the openings of the through-holes on the first surface of the absorbent core have a maximum inner diameter that is 0.2 to 0.8 times the width of the lengthwise groove section.

Since the openings of the through-holes of the absorbent article of aspect 5 have a maximum inner diameter that is 0.2 to 0.8 times the width of the lengthwise groove section, there is less likelihood of the absorbing material (SAP or the like) in the absorbent core falling down from the through-holes or of the strength of the absorbent core being reduced due to the presence of the through-holes, while excreted fluid that has been discharged from the wearer can migrate smoothly in the thickness direction via the through-holes.

The absorbent article according to another aspect (aspect 6) of the invention is the absorbent article of aspect 5 wherein the plurality of through-holes are arranged in the lengthwise groove section with intervals that are longer than the maximum inner diameter of the openings.

Since the through-holes of the absorbent article of aspect 6 are arranged in the lengthwise groove section with intervals that are longer than maximum inner diameter of the openings, excreted fluid that has reached the first surface of the absorbent core can enter into the through-holes while spreading over the first surface, and since the through-holes through which the excreted fluid passes are less likely to be maldistributed, excreted fluid can be absorbed through a wide range of locations of the absorbent core. In addition, since the through-holes are provided with a minimum fixed interval between them, the strength of the absorbent core is less likely to be reduced, and the absorption property of the absorbent body (especially the diffusibility and absorption efficiency) can be improved in a stable manner.

The absorbent article according to yet another aspect (aspect 7) of the invention is the absorbent article of any of aspects 1 to 6 wherein the absorbent body is partitioned into one edge area, another edge area and a center area between them, by imaginary lines that divide the absorbent body into three equal portions in the widthwise direction, and the absorbent core comprises the lengthwise groove section in all of the areas including the one edge area, the other edge area and the center area.

Since the absorbent article of aspect 7 has the lengthwise groove section provided spanning across the entire width in the widthwise direction of the absorbent body, excreted fluid can be absorbed through a wide region of the absorbent core, and a more excellent absorption property (especially absorption efficiency) and rewetting property as an absorbent article can be exhibited. Furthermore, since the lengthwise groove section is formed in the one edge area and the other edge area in the widthwise direction of the absorbent core, an advantage is provided in that excreted fluid is less likely to leak from both edges in the widthwise direction of the absorbent article (i.e., in the area surrounding the legs of the wearer), via both edges in the widthwise direction of the absorbent body.

The absorbent article according to yet another aspect (aspect 8) of the invention is the absorbent article of any of aspects 1 to 7 wherein the absorbent core comprises a widthwise groove section that is a groove section indented from the second surface and extending in the widthwise direction, and having lower basis weight than the average basis weight of the absorbent core.

Since the absorbent core of the absorbent article of aspect 8 comprises the widthwise groove section on the second surface, excreted fluid that has been supplied to the first surface of the absorbent core flows into the widthwise groove section via the through-holes and the lengthwise groove section, also flowing through the widthwise groove section and diffusing in the widthwise direction of the absorbent body, and therefore excreted fluid that has been discharged from the wearer can be absorbed into the absorbent core through a wide region of the absorbent core. As a result, it is possible to exhibit an even more excellent absorption property and rewetting property as an absorbent article.

The absorbent article according to yet another aspect (aspect 9) of the invention is the absorbent article of aspect 8 wherein the widthwise groove section is provided intermittently in the widthwise direction on the second surface of the absorbent core.

Since the widthwise groove section of the absorbent article of aspect 9 is provided intermittently in the widthwise direction, when excreted fluid reaches the widthwise groove section of the absorbent core and diffuses in the widthwise direction, the excreted fluid is readily absorbed into the absorbent core by capillary movement through the wall sections of the absorbent core faced by the widthwise edges of the partitioned widthwise groove section, thereby allowing the absorption property (especially the absorption rate) and the rewetting property of the absorbent body to be even further improved.

The absorbent article according to yet another aspect (aspect 10) of the invention is the absorbent article of aspect 8 or 9 wherein the absorbent article is partitioned into three regions in the lengthwise direction: the front waist region, the rear waist region and the crotch region between them, the absorbent body being disposed across the three regions and the absorbent core comprising the widthwise groove section in all of the three regions.

Since the absorbent body of the absorbent article of aspect 10 comprises the widthwise groove section in all of the regions in the lengthwise direction of the absorbent article, excreted fluid that has been discharged from the wearer can be absorbed through a wide region in the lengthwise direction and the widthwise direction of the absorbent core, and an even more excellent absorption property and rewetting property as an absorbent article can be exhibited.

The absorbent article according to yet another aspect (aspect 11) of the invention is the absorbent article of any one of aspects 8 to 10 wherein the core wrap is not joined to the bottom part of the widthwise groove section at a section of the widthwise groove section that is not crossing with the lengthwise groove section.

Since the core wrap of the absorbent article of aspect 11 is not joined to the bottom part of the widthwise groove section at the section of the widthwise groove section that is not crossing with the lengthwise groove section, even though the widthwise groove section that has absorbed excreted fluid flowing through it collapse by expansion of the absorbent core, since the lengthwise groove section and the widthwise groove section that excreted fluid has not reached do not lose their function as excreted fluid diffusion channels (even when the lengthwise groove section has absorbed excreted fluid, it is able to ensure the fixed space running in the lengthwise direction as mentioned above, and therefore its function as an excreted fluid diffusion channel is not lost), the excreted fluid of further subsequent excretions can flow through the lengthwise groove section and diffuse to the non-collapsed widthwise groove section. Since this process is repeated when excreted fluid is supplied several times, the excreted fluid can be absorbed over a wide region of the absorbent body.

Consequently, the absorbent article of aspect 11 can exhibit an even more excellent absorption property and rewetting property particularly for multiple excretions, and the absorbent article can be worn for prolonged periods.

The absorbent article according to yet another aspect (aspect 12) of the invention is the absorbent article of any one of aspects 1 to 11 which comprises a diffusion sheet between the core wrap disposed on the second surface side of the absorbent core and the liquid-impermeable sheet, the diffusion sheet being joined with the core wrap disposed on the second surface side of the absorbent core, at the section that is joined to the bottom part of the lengthwise groove section.

Since the diffusion sheet of the absorbent article of aspect 12 is joined with the core wrap that is disposed on the second surface side of the absorbent core, at the section that is joined to the bottom part of the lengthwise groove section, even when the absorbent body has absorbed excreted fluid and the absorbent core has expanded, the fact that the core wrap and the diffusion sheet lie between the pair of side walls that have swelled in the widthwise direction of the lengthwise groove section makes it possible to more reliably ensure the fixed space running in the lengthwise direction inside the lengthwise groove section. As a result, the absorbent article of aspect 12 can more reliably maintain the function of the lengthwise groove section as an origin for folding and its function as the excreted fluid diffusion channel.

In addition, since excreted fluid that has reached the lengthwise groove section is easily diffused in the lengthwise direction of the absorbent body by the lengthwise groove section and the diffusion sheet, it is possible to more rapidly cause diffusion of the excreted fluid in the lengthwise direction of the absorbent body while also allowing absorption over a wide region in the lengthwise direction.

Consequently, the absorbent article of aspect 12 has flexibility and an excellent fitting property, excellent diffusibility in the lengthwise direction and an excellent rewetting property even after absorption of excreted fluid, and can also exhibit these effects more reliably.

The absorbent article according to yet another aspect (aspect 13) of the invention is the absorbent article of aspect 12 wherein the diffusion sheet is a layered sheet including a first fiber layer containing hydrophilic fibers with a mean fiber length of 25 mm to 64 mm, a second fiber layer containing hydrophilic fibers with a mean fiber length of 25 mm to 64 mm, and a pulp-containing pulp fiber layer between them.

Since the diffusion sheet having the specific structure described above has excellent performance for causing diffusion of excreted fluid in the in-plane direction of the sheet, excreted fluid that has reached the diffusion sheet of the absorbent article of aspect 13 easily diffuses over a wide region in the lengthwise direction of the absorbent body, and the excreted fluid can be absorbed over a wide region of the absorbent body. Consequently, the absorbent article of aspect 13 can exhibit an even more excellent absorption property and rewetting property as an absorbent article.

The absorbent article according to yet another aspect (aspect 14) of the invention is the absorbent article of aspect 12 or 13 wherein the core wrap and the diffusion sheet each have holes that communicate with the through-holes of the lengthwise groove section.

Since the absorbent article of aspect 14 has holes formed in both the core wrap and the diffusion sheet that communicate with the through-holes of the lengthwise groove section, excreted fluid that has been supplied to the absorbent body easily reaches the second surface side of the absorbent body via the holes and through-holes, allowing the absorption property (especially the absorption rate) in the thickness direction of the absorbent body to be even further improved, and allowing the effect of aspect 12 or 13 to be exhibited even more.

The absorbent article according to yet another aspect (aspect 15) of the invention is the absorbent article of any one of aspects 12 to 14 wherein the diffusion sheet has a plurality of slits running through the thickness direction.

When the core wrap and diffusion sheet are joined to the bottom part of the lengthwise groove section of the absorbent body, i.e. when they are embedded in the lengthwise groove section, the rigidity of the lengthwise groove section of the absorbent body tends to increase, particularly after the absorbent body has repeatedly absorbed excreted fluid, but since the diffusion sheet of the absorbent article of aspect 15 has a plurality of slits, the rigidity of the diffusion sheet itself is reduced, allowing increase in the rigidity of the absorbent body to be prevented. Therefore, the absorbent article of aspect 15 has flexibility despite the presence of the diffusion sheet, and can exhibit an excellent fitting property.

The absorbent article according to yet another aspect (aspect 16) of the invention is the absorbent article of any one of aspects 1 to 15 wherein the region that includes the absorbent body has a flexural rigidity value of 5.0 to 35.0 mN·m in the lengthwise direction and the widthwise direction.

Since the absorbent article of aspect 16 has a prescribed flexural rigidity in the region including the absorbent body, it is possible to obtain a more excellent fitting property as an absorbent article.

### Advantageous Effects of Invention

According to the invention it is possible to provide an absorbent article having an excellent fitting property and also an excellent rewetting property and feel during wear, not only before absorption but also after absorption of excreted fluid such as urine.

### Brief Description of the Drawings

Fig. 1 is a plan view of an absorbent article 1 according to a first embodiment of the invention.
Fig. 2 is a plan view of the absorbent body 7 of the absorbent article 1 according to the first embodiment of the invention, as viewed from the second surface side.
Fig. 3 is a plan view of the absorbent core 9 of the absorbent article 1 according to the first embodiment of the invention, as viewed from the second surface side.
Fig. 4 is a perspective view schematically showing the absorbent body 7 and absorbent core 9 of the absorbent article 1 according to the first embodiment of the invention.
Fig. 5 is a partial schematic magnified end view along line V-V of Fig. 2.
Fig. 6 is a partial schematic magnified end view along line VI-VI of Fig. 2.
Fig. 7 is a partial schematic magnified end view along line VII-VII of Fig. 2.
Fig. 8 is a partial schematic magnified end view along line VIII-VIII of Fig. 2.
Fig. 9 is a schematic diagram illustrating absorption of excreted fluid by the absorbent article 1 of the first embodiment of the invention.
Fig. 10 is a partial schematic magnified end view along line X-X of Fig. 9.
Fig. 11 is a diagram illustrating an absorbent article having no compressed sections in the lengthwise groove sections.
Fig. 12 is a plan view of the absorbent core 9 of the absorbent article according to another embodiment of the invention, as viewed from the second surface side.
Fig. 13 is a plan view of a diffusion sheet 13 having a plurality of slits 41 in the lengthwise direction.

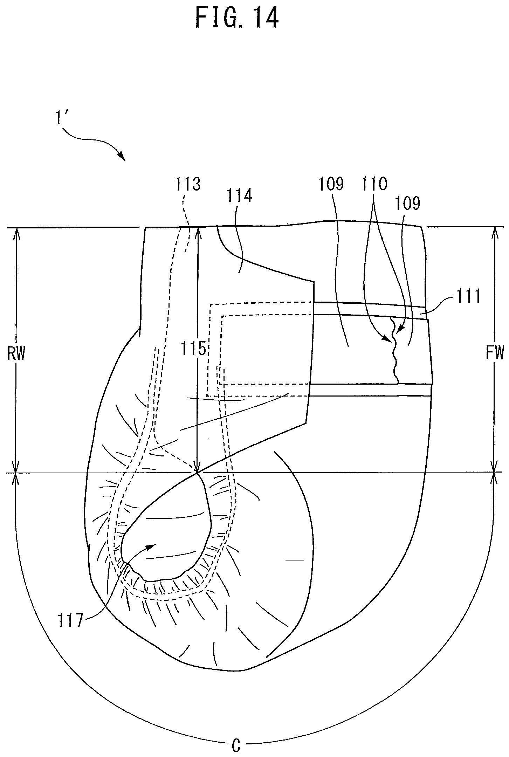

Fig. 14 is a diagram illustrating the front waist region, the rear waist region and the crotch region of a tape-type disposable diaper.

### Description of Embodiments

### [Definitions]

### [Fitting property]

Throughout the present description, the property of the absorbent article of following (fitting to) the bodily form of the wearer is referred to as the "fitting property".

### [Rewetting property]

Throughout the present description, the property by which excreted fluid absorbed into the absorbent body is unlikely to return to the wearer side from the liquid-permeable sheet is referred to as an "excellent rewetting property".

### [Lengthwise direction and short direction]

Throughout the present description, the terms "lengthwise direction, "widthwise direction" and thickness direction", as they relate to the absorbent body, mean the direction of the lengthwise axis line of the absorbent body, the direction of the axis line perpendicular to the lengthwise axis line (hereunder also referred to as "widthwise axis line"), and the direction of the axis line perpendicular to both the lengthwise axis line and the widthwise axis line, respectively.

### [Lengthwise groove section and its width]

Throughout the present description, the term "lengthwise groove section" running in the lengthwise direction means not only a groove section running in a direction parallel to the lengthwise axis line, but also a groove section running essentially in the lengthwise axis line direction. That is, a lengthwise groove section may run in a straight linear or non-linear (for example, curved) manner in the lengthwise direction.

Throughout the present description, the term "width of the lengthwise groove section" means the length of the lengthwise groove section in the direction perpendicular to the direction in which the lengthwise groove section runs at the section of interest.

### [Widthwise groove section and its width]

Throughout the present description, the term "widthwise groove section" running in the widthwise direction means not only a groove section running in a direction parallel to the widthwise axis line, but also a groove section running in a direction essentially parallel to the widthwise direction. That is, a widthwise groove section may run in a straight linear or non-linear (for example, curved) manner in the widthwise direction.

Throughout the present description, the term "width of the widthwise groove section" means the length of the widthwise groove section in the direction perpendicular to the direction in which the widthwise groove section runs at the section of interest.

### [Front waist region, rear waist region and crotch region]

Throughout the present description, the terms "front waist region", "rear waist region" and "crotch region" are terms used for when the absorbent article is a disposable diaper, and they have the following meanings.

### [Pants-type disposable diaper]

For a pants-type disposable diaper, the front waist region is the region among the front body region that is sandwiched by a pair of joining sections joining the front body region and the rear body region, while the rear waist region is the region among the rear body region that is sandwiched by the aforementioned pair of joining sections.

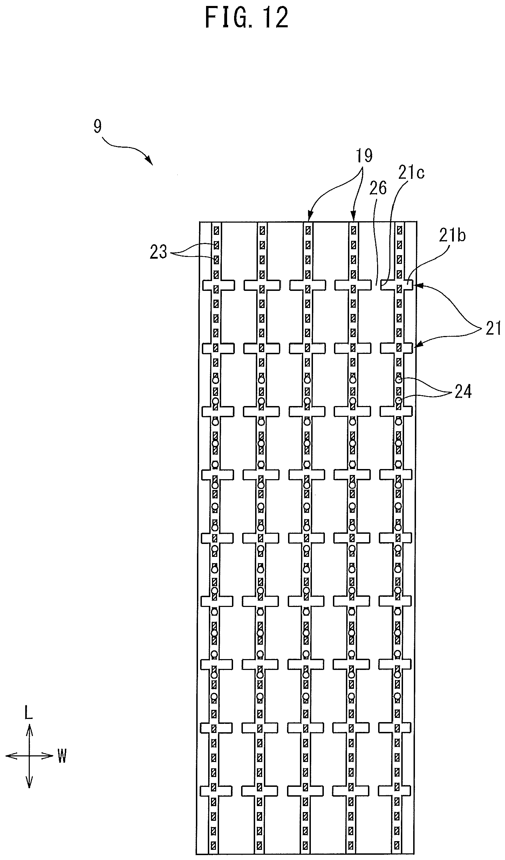

Also, for a pants-type disposable diaper, the crotch region is the region between the front waist region and the rear waist region. The crotch region also corresponds to the region sandwiched by a pair of leg-surrounding openings (numeral 117 in Fig. 12).

### [Tape-type disposable diaper]

For a tape-type disposable diaper, as shown in Fig. 12, the waist region and crotch region are partitioned with the tip ends 110 of a pair of tape fasteners 109 being anchored adjacent to each other in a predetermined tape fastener anchoring region 111.

Specifically, the waist region (FW + RW) is judged based on a pair of overlapping sections 115 where the waist-forming member 113 of the front body region and the waist-forming member 114 of the rear body region of the disposable diaper 1' overlap, in the anchored state described above. The front waist region FW is the region of the front body region of the disposable diaper 1 that is between the pair of overlapping sections 115. Similarly, the rear waist region RW is the region of the rear body region of the disposable diaper 1' that is between the pair of overlapping sections 115. The crotch region (C) is the region between the front waist region FW and the rear waist region RW.

Throughout the present description, unless otherwise specified, the simple phrase "planar view" will be used to mean the view of an object in the deployed flat state from the upper side in the thickness direction.

Preferred embodiments of the absorbent article of the invention will now be described in detail with reference to the accompanying drawings.

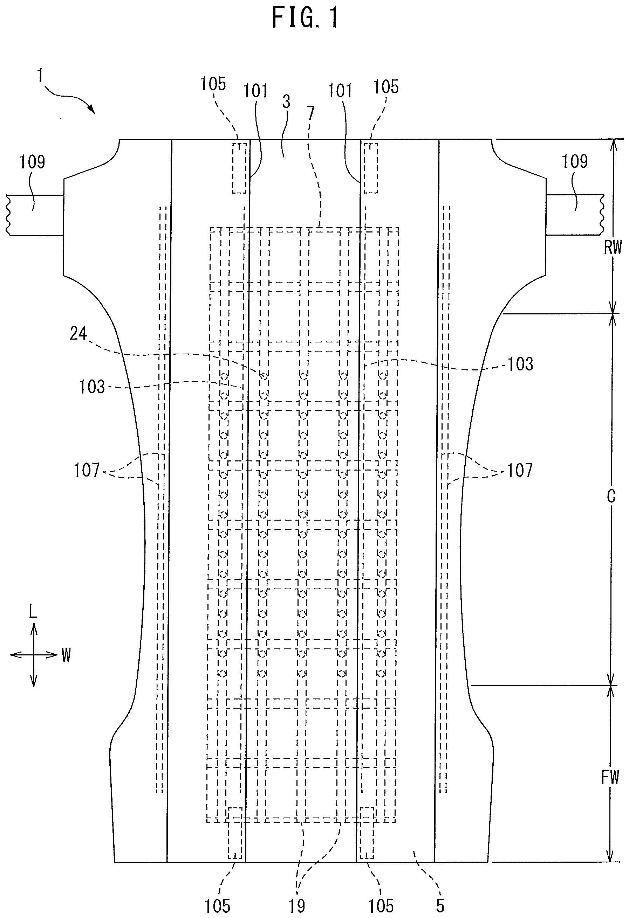

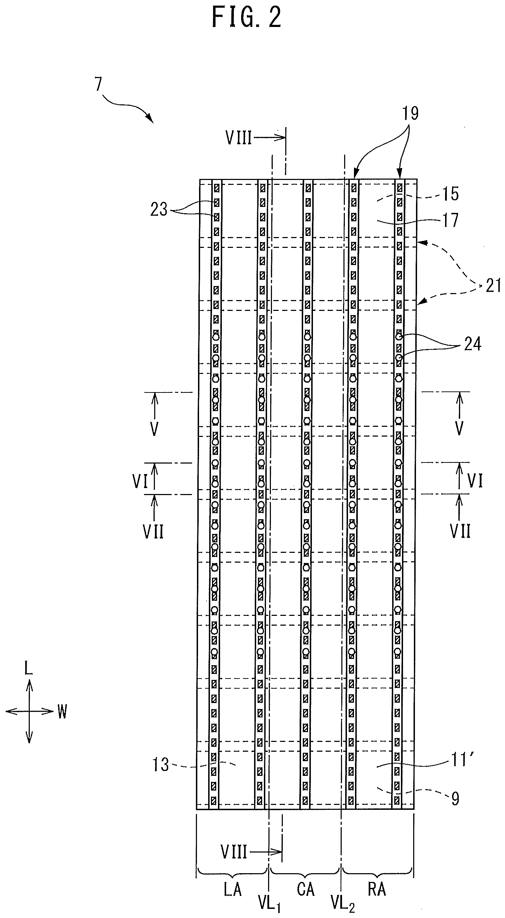

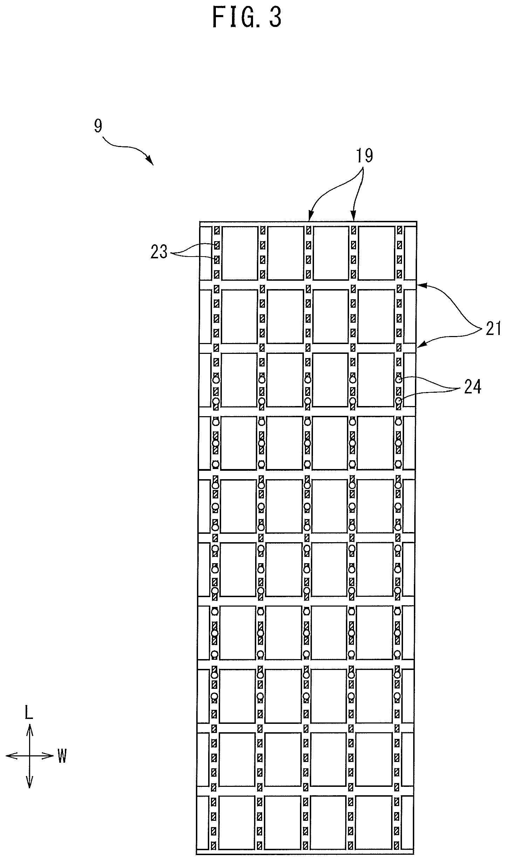

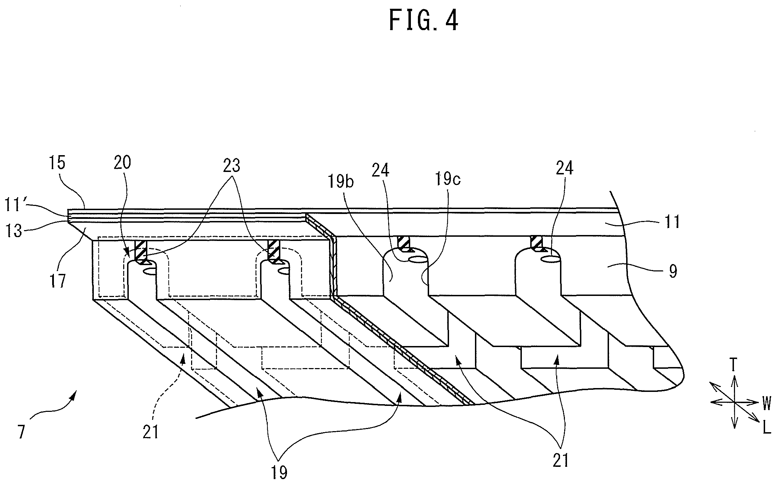

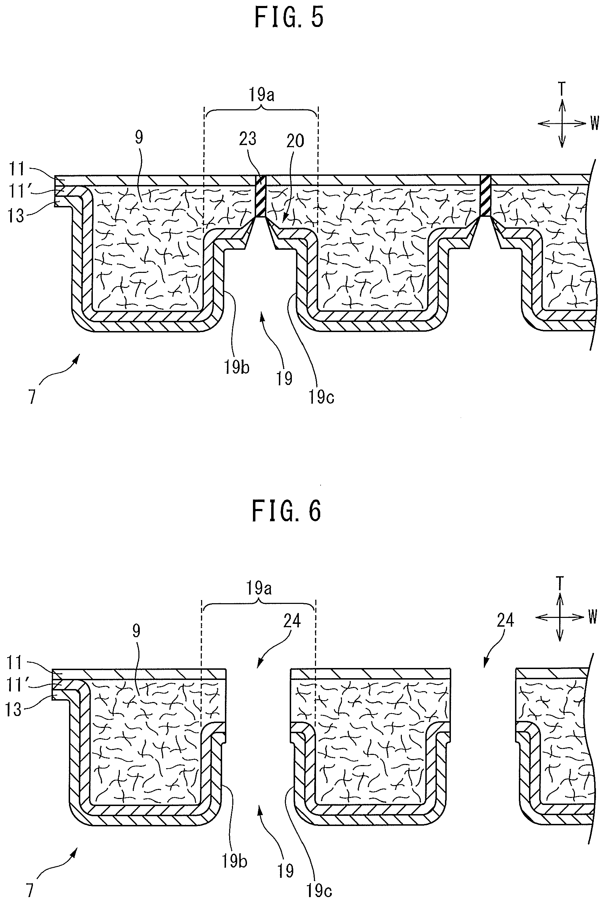

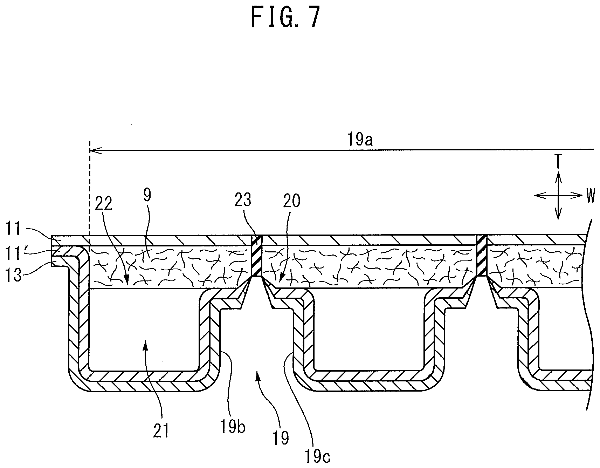

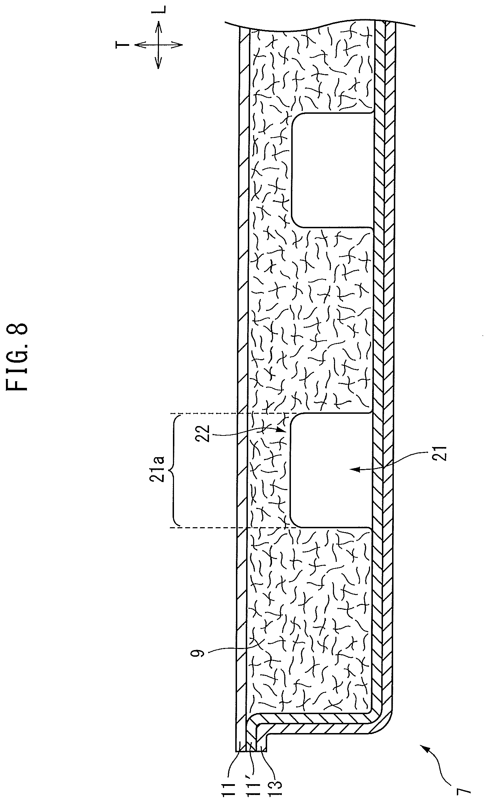

Fig. 1 to Fig. 8 are diagrams illustrating an absorbent article 1, and specifically a tape-type disposable diaper, according to the first embodiment of the invention. Specifically, Fig. 1 is a plan view of the absorbent article 1. Fig. 2 is a plan view of the absorbent body 7 of the absorbent article 1, as viewed from the second surface side. Fig. 3 is a plan view of the absorbent core 9 of the absorbent article 1, as viewed from the second surface side. Fig. 4 is a perspective view schematically showing the absorbent body 7 and absorbent core 9 of the absorbent article 1. Fig. 5 is a partial schematic magnified end view along line V-V of Fig. 2. Fig. 6 is a partial schematic magnified end view along line VI-VI of Fig. 2. Fig. 7 is a partial schematic magnified end view along line VII-VII of Fig. 2. Fig. 8 is a partial schematic magnified end view along line VIII-VIII of Fig. 2.

As shown in Figs. 1 to 8, the absorbent article 1 of the first embodiment has a lengthwise direction L, a widthwise direction W and a thickness direction T that are mutually perpendicular, and it comprises a liquid-permeable sheet 3, a liquid-impermeable sheet 5, an absorbent body 7 between the liquid-permeable sheet 3 and liquid-impermeable sheet 5, and a diffusion sheet 13 between the absorbent body 7 and liquid-impermeable sheet 5.

The absorbent body 7 has a first surface facing the liquid-permeable sheet and a second surface facing the liquid-impermeable sheet, and further comprises an absorbent core 9 having a skin side surface 15 (first surface) and a clothing side surface 17 (second surface), a core wrap 11 covering the skin side surface 15 of the absorbent core 9, and a core wrap 11' covering the clothing side surface 17 of the absorbent core 9.

Also, the absorbent core 9 comprises lengthwise groove sections 19 which are groove sections depressed in the thickness direction T of the absorbent core 9 from the clothing side surface 17 and running in the lengthwise direction L, and comprising low-basis-weight regions 19a having lower basis weight than the average basis weight of the absorbent core 9, and widthwise groove sections 21 which are groove sections depressed in the thickness direction T from the clothing side surface 17 and running in the widthwise direction W, and comprising low-basis-weight regions 21a having lower basis weight than the average basis weight of the absorbent core 9.

Incidentally, as shown in Figs. 2 to 4, a plurality of lengthwise groove sections 19 and widthwise groove sections 21 are each disposed on the clothing side surface 17 of the absorbent core 9, the plurality of widthwise groove sections 21 being disposed so as to cross respectively with the plurality of lengthwise groove sections 19, and connect with them.

As shown in Figs. 2 to 7, the lengthwise groove sections 19 include compressed sections 23 formed by intermittent compressing along the lengthwise groove sections 19, at parts of the lengthwise groove sections 19 in the widthwise direction W, in the thickness direction of the absorbent body, and a plurality of through-holes 24 running from the skin side surface 15 through to the clothing side surface 17, and in the lengthwise groove sections 19, the core wrap 11' is joined to the absorbent core 9 at the bottom parts 20 of the lengthwise groove sections 19. For this embodiment, the diffusion sheet 13 is joined with the core wrap 11' in the lengthwise groove sections 19, and more specifically with the core wrap 11' at the portions that are joined to the bottom parts 20 of the lengthwise groove sections 19.

The compressed sections 23 are formed by simultaneously compressing the two core wraps 11, 11' covering the absorbent core 9, the absorbent core 9, and the diffusion sheet 13, inside the lengthwise groove sections 19.

Also, the through-holes 24 are formed by perforating the two core wraps 11, 11', the absorbent core 9 and the diffusion sheet 13 inside the lengthwise groove sections 19, using any desired means such as a circular blade or a needle.

As shown in Fig. 2, the absorbent body 7 is partitioned into a right area RA, a left area LA and a center area CA between the right area RA and left area LA, by imaginary lines VL₁ and VL₂ that partition the absorbent body 7 into three equal portions in the widthwise direction W, the absorbent body 7 comprising lengthwise groove sections 19 in all of the areas including the right area RA, left area LA and center area CA.

As shown in Fig. 1, the absorbent article 1 of the first embodiment is partitioned into three regions in the lengthwise direction L: a front waist region RW, a rear waist region LW and a crotch region C between the front waist region RW and rear waist region LW, the absorbent body 7 being disposed across the three regions. The absorbent body 7 also comprises widthwise groove sections 21 in all of the three regions.

Furthermore, as shown in Fig. 4, Fig. 7 and Fig. 8, according to the first embodiment, the core wrap 11' and diffusion sheet 13 are not joined to the bottom parts 22 of the widthwise groove sections 21 at the sections of the widthwise groove sections 21 that are not crossing with the lengthwise groove sections 19. That is, the sections of the widthwise groove sections that are not crossing with the lengthwise groove sections 19 are formed between the absorbent core 9 and the core wrap 11' and diffusion sheet 13.

Incidentally, according to the first embodiment, the absorbent article 1 has a pair of anti-leakage walls 101 including an elastic member 103, an anchoring part 105 for anchoring to the liquid-permeable sheet 3, an elastic member 107 and a tape fastener 109, as shown in Fig. 1, but because these are known in the technical field they will not be explained here.

The excellent fitting property and excellent rewetting property and feel during wear of the absorbent article of the invention, both before and after absorption of excreted fluid, will now be explained using the first embodiment.

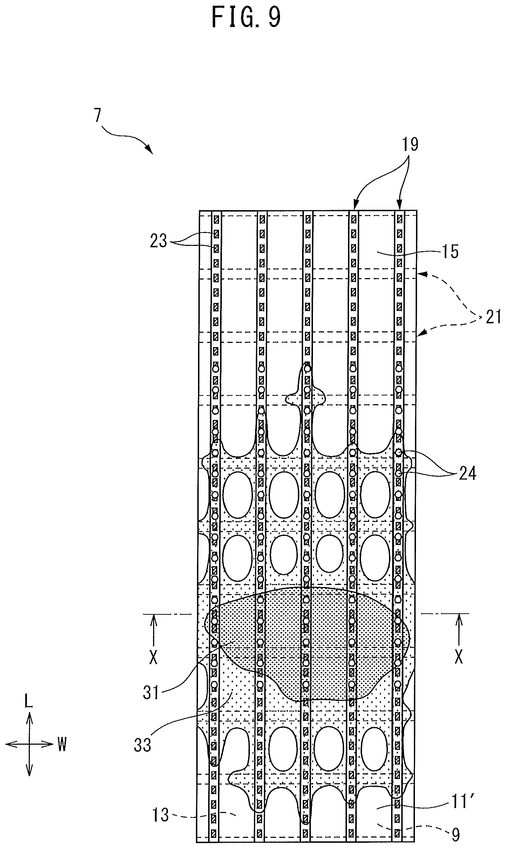

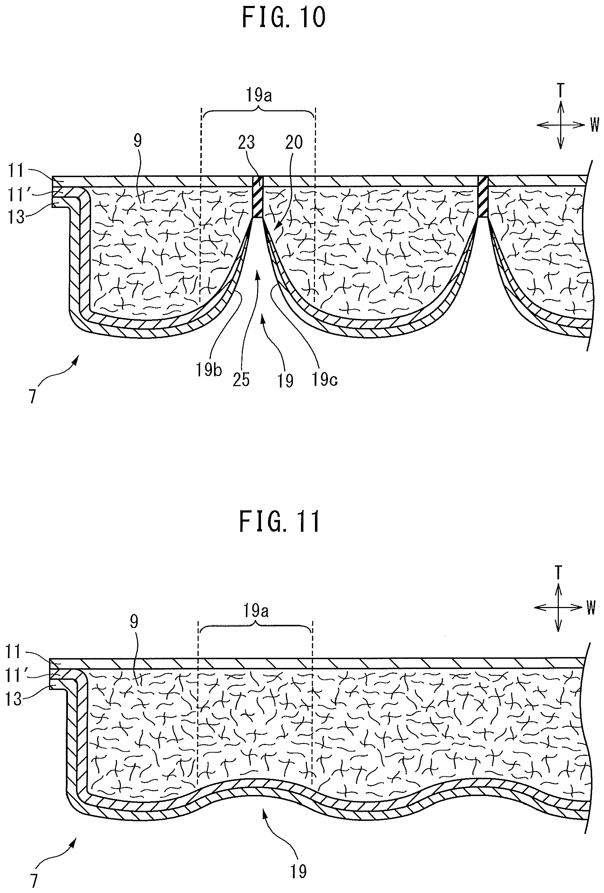

Fig. 9 is a schematic diagram illustrating absorption of excreted fluid by the absorbent article 1 of the first embodiment of the invention, and it corresponds to Fig. 2. Fig. 10 is an end view along line X-X of Fig. 9.

First, excreted fluid such as urine that has been discharged from the wearer permeates the liquid-permeable sheet 3 and reaches the first surface of the absorbent body 7. Excreted fluid that has reached the first surface of the absorbent body 7 penetrates in the thickness direction T of the absorbent body 7 while spreading over the first surface of the absorbent body 7, and is absorbed into the absorbent core 9. During this time, some of the excreted fluid flows into the through-holes 24 where openings are present in the first surface of the absorbent body 7, and reaches the second surface side of the absorbent body 7 through the through-holes 24. Even when passing through the through-holes 24, some of the excreted fluid is absorbed in the absorbent core 9 through the inner walls of the through-holes 24.

Moreover, as shown in Fig. 9, the excreted fluid 31 that has reached the second surface of the absorbent body 7 forms excreted fluid 33 diffused on the second surface of the absorbent body 7, as explained below.
(i) First, the excreted fluid 31 that has reached the second surface of the absorbent body 7 flows in the lengthwise groove sections 19 formed on the second surface of the absorbent body 7, and diffuses in the lengthwise direction L of the absorbent body 7. During this time, while the excreted fluid 31 is flowing through the lengthwise groove sections 19, a portion thereof passes through the core wrap 11' and diffusion sheet 13, permeating into the interior of the absorbent body 7 (i.e., the absorbent core 9).
(ii) A portion of the excreted fluid 31 that has diffused in the lengthwise direction L then passes from the lengthwise groove sections 19 through the core wrap 11', flowing into the widthwise groove sections 21 formed between the absorbent core 9 and the core wrap 11' and diffusion sheet 13, and diffusing in the widthwise direction W of the absorbent body 7.
(iii) The excreted fluid 31 that has diffused in the lengthwise direction L and widthwise direction W in this manner passes through the core wrap 11' and diffusion sheet 13 in the thickness direction T, permeating into the absorbent body 7 (i.e., the absorbent core 9).
(iv) The excreted fluid 31 that has diffused in the lengthwise direction L and widthwise direction W then diffuses into the absorbent core 9, forming the aforementioned diffused excreted fluid 33.

As shown in Figs. 2 to 7, the lengthwise groove sections 19 are composed of low-basis-weight regions 19a, and they have compressed sections 23 formed by compressing the diffusion sheet 13, the two core wraps 11, 11' and the absorbent core 9, and a plurality of through-holes 24 formed by perforating the diffusion sheet 13, the two core wraps 11, 11' and the absorbent core 9, and running from the skin side surface 15 through to the clothing side surface 17.

Since the absorbent core 9 of the absorbent body 7 in the absorbent article 1 thus has lengthwise groove sections 19 comprising a plurality of through-holes 24, excreted fluid supplied to the skin side surface 15 of the absorbent body 7 (particularly repeatedly supplied excreted fluid) can rapidly penetrate in the thickness direction T of the absorbent core 9 via the through-holes 24.

Thus, since the absorbent article 1 can rapidly supply excreted fluid to the excreted fluid diffusion channels formed by the lengthwise groove sections 19, it is possible to obtain an excellent absorption property (particularly absorption rate and diffusibility) and the resulting excellent rewetting property, as an absorbent article.

Moreover, excreted fluid that has reached the lengthwise groove sections 19 of the absorbent body 7 is diffused in the lengthwise direction L along the lengthwise groove sections 19, while being absorbed from the side walls of the lengthwise groove sections 19 through the diffusion sheet 13 and core wrap 11' into the absorbent core 9, and therefore the excreted fluid can be absorbed from a wide region in the lengthwise direction L of the absorbent body 7, and a more excellent absorption property (particularly absorption efficiency) and rewetting property can be obtained for the absorbent article.

In addition, since the absorbent article 1 has the core wrap 11' joined to the compressed sections 23 at the bottom parts of the lengthwise groove sections 19, even when the absorbent core 9 has absorbed excreted fluid and expanded, expansion of the absorbent core 9 by the compressed sections 23 is inhibited at the bottom parts of the lengthwise groove sections 19, while at the pair of side walls 19b, 19c running along the lengthwise direction L of the lengthwise groove sections 19, even though the pair of side walls 19b, 19c are swelled in the widthwise direction W and deform in a manner that would plug the lengthwise groove sections 19, since the core wrap 11' and diffusion sheet 13 that are joined to the bottom parts of the lengthwise groove sections 19 stop inside the lengthwise groove sections 19 and lie between the pair of side walls 19b, 19c that have swelled in the widthwise direction W, fixed spaces 25 can be ensured inside the lengthwise groove sections 19 running in the lengthwise direction L, as shown in Fig. 10. When such spaces 25 are ensured inside the lengthwise groove sections 19, the function of the lengthwise groove sections 19 as origins for folding and their function as excreted fluid diffusion channels can be exhibited, and therefore even after absorption of excreted fluid (especially after repeated absorption of excreted fluid), the absorbent article 1 has flexibility and an excellent fitting property, while also being able to retain the aforementioned excellent absorption property and rewetting property.

In particular, since the lengthwise groove sections 19 can ensure the fixed spaces 25 running in the lengthwise direction L inside the lengthwise groove sections 19 even when the absorbent article 1 has repeatedly absorbed excreted fluid, excreted fluid can be absorbed over a wide region in the lengthwise direction L of the absorbent body 7, and as a result, excreted fluid absorbed into the absorbent body 7 of the absorbent article 1 is less likely to return to the front surface of the liquid-permeable sheet 3, and an excellent rewetting property can be exhibited.

Consequently, the absorbent article 1 has an excellent fitting property and absorption property, as well as an excellent rewetting property, not only before absorption but also after absorption of excreted fluid, and therefore a satisfactory feel during wear can be obtained for prolonged periods.

The significance of the compressed sections in the lengthwise groove sections will now be explained. Fig. 11 is a diagram illustrating an absorbent article having no compressed sections in the lengthwise groove sections. Fig. 11 is an end view corresponding to the end face along line X-X of Fig. 9. The absorbent article shown in Fig. 11 has the same construction as the absorbent article 1 of the first embodiment, except that it does not have compressed sections in the lengthwise groove sections.

In an absorbent article having no compressed sections in the lengthwise groove sections 19, as shown in Fig. 11, when the absorbent body 7 absorbs excreted fluid, the pair of side walls 19b, 19c of the lengthwise groove sections 19 swell in the widthwise direction W and plug the lengthwise groove sections 19, such that excreted fluid that has reached the clothing side surface 17 of the absorbent body 7 is less able to flow inside the lengthwise groove sections 19, and it may not be possible to adequately diffuse the excreted fluid in the lengthwise direction L of the absorbent body 7. In addition, when the lengthwise groove sections 19 have almost disappeared by expansion of the absorbent body, the lengthwise groove sections 19 fail to function as origins for folding, and therefore the absorbent article exhibits a poorer fitting property as excreted fluid is absorbed.

According to the first embodiment described above, the lengthwise groove sections 19 are formed only on the clothing side surface 17 of the absorbent core 9; however, in the absorbent article of the invention the lengthwise groove sections may be formed on both surfaces, the skin side surface and clothing side surface, of the absorbent core. When the lengthwise groove sections are formed on both surfaces of the absorbent core, it is possible to further improve the diffusibility of excreted fluid in the lengthwise direction.

Also, according to the first embodiment, the widthwise groove sections 21 are formed on the clothing side surface 17 of the absorbent core 9; however, in the absorbent article of the invention the widthwise groove sections may be formed on either surface, the skin side surface or the clothing side surface of the absorbent core, or they may be formed on both surfaces.

However, when the widthwise groove sections are provided on the same surface as the lengthwise groove sections (i.e., when the widthwise groove sections are provided on at least the clothing side surface of the absorbent core), excreted fluid supplied to the first surface (skin side surface) of the absorbent core will flow through the through-holes and lengthwise groove sections and into the widthwise groove sections, while flowing through the widthwise groove sections and diffusing in the widthwise direction of the absorbent body, such that excreted fluid discharged by the wearer can be absorbed over a wider region of the absorbent core. As a result, it is possible to exhibit an even more excellent absorption property and rewetting property as an absorbent article.

As shown in Fig. 2 and Fig. 3, according to the first embodiment, the widthwise groove sections 21 are provided continuously in the widthwise direction W of the absorbent article 1; however according to the invention there is no limitation to this aspect, and for example, the widthwise groove sections 21 may be provided intermittently in the widthwise direction W as according to another embodiment of the invention illustrated in Fig. 12. If the widthwise groove sections 21 are provided intermittently in the widthwise direction W, then when excreted fluid reaches the widthwise groove sections 21 of the absorbent core 9 and diffuses in the widthwise direction W, the excreted fluid will be easily absorbed into the absorbent core 9 by capillary movement through the wall sections 26 of the absorbent core 9 faced by the widthwise edges 21c of the partitioned widthwise groove sections 21b, thereby allowing the absorption property (especially the absorption rate) and the rewetting property of the absorbent body 7 to be even further improved.

Furthermore, according to the first embodiment described above, the lengthwise groove sections 19 includes compressed sections 23 formed by compressing part of the widths of the lengthwise groove sections 19 intermittently along the lengthwise groove sections 19. By forming compressed sections in this manner, the wearer is less likely to feel the hardness of the compressed sections. In an absorbent article according to another embodiment of the invention, the compressed sections may be formed by compressing part of the widths of the lengthwise groove sections continuously in the lengthwise direction. By forming compressed sections in this manner, the absorbent article will easily fold on the lengthwise groove sections as origins, and will have an excellent fitting property.

In the first embodiment of the invention, as shown in Fig. 1 to Fig. 4 and Fig. 6, the lengthwise groove sections 19 include a plurality of through-holes 24 that are formed by perforating the diffusion sheet 13, the two core wraps 11, 11' and the absorbent core 9, and that run from the skin side surface 15 through to the clothing side surface 17. When a plurality of through-holes are thus provided in the lengthwise groove sections, excreted fluid supplied to the skin side surface of the absorbent body can rapidly pass through the plurality of through-holes to the clothing side surface of the absorbent body, and therefore within a short period of time after excreted fluid has been discharged, it is possible to diffuse the excreted fluid in the lengthwise direction by the lengthwise groove sections formed on the clothing side surface of the absorbent body. Consequently, the absorbent article of this embodiment can exhibit an excellent absorption property (especially absorption rate and diffusibility), and consequently an excellent rewetting property.

According to the invention, the structures of the through-holes (for example, the shapes and sizes of the openings) are not particularly restricted, and they may be appropriately set depending on the absorption property (for example, absorption rate), the absorbent body strength, and flexibility. For example, the shapes of the openings of the through-holes (hereunder referred to simply as "openings") in the planar view (hereunder referred to simply as the "shapes of the openings") may be circular shapes (including ellipsoid shapes), or rectangular shapes, or other geometrical shapes such as star shapes.

In addition, the sizes of the through-holes are not particularly restricted so long as they are sizes allowing the through-holes to be formed in the lengthwise groove sections, but from the viewpoint of the absorption property and strength, the maximum inner diameter of the openings will usually be in the range of 1 mm to 5 mm, and is preferably in the range of 2 mm to 4 mm. The term "maximum inner diameter of the openings" means the inner diameter having the longest length among the inner diameters of the openings (for example, the diameter, if the shapes of the openings are circular; the long diameter, if they are elliptical; or the long diagonal, if they are quadrilateral). When the shapes of the openings are circular shapes, for example, the maximum inner diameter (i.e. diameter) will usually be in the range of 1 mm to 5 mm and is preferably in the range of 2 mm to 4 mm. Throughout the present description, "circular" includes not only "perfectly circular" but also "circular" in the wide sense including elliptical shapes or distorted circular shapes that may result during production steps.

According to the invention, the through-holes preferably have openings with a maximum inner diameter of 0.2 to 0.8 times the widths of the lengthwise groove sections. If the openings of the through-holes have such maximum inner diameters, there will be less of a chance for the absorbing material (SAP or the like) in the absorbent core to fall out through the through-holes, or for the strength of the absorbent core to be reduced by the presence of the through-holes, while excreted fluid supplied to the skin side surface of the absorbent core will be able to migrate smoothly in the thickness direction.

According to the invention, the plurality of through-holes are preferably arranged in the lengthwise groove sections at intervals that are longer than the maximum inner diameter of the openings. If the through-holes are thus arranged in the lengthwise groove sections at intervals that are longer than the maximum inner diameter of the openings, excreted fluid supplied to the skin side surface of the absorbent core will readily flow into the through-holes while spreading over the skin side surface of the absorbent core, so that maldistribution will be less likely to occur in the through-holes into which the excreted fluid flows (i.e., the excreted fluid will more easily flow into more through-holes), and therefore the excreted fluid can be made to migrate to the clothing side surface from a wider range of locations of the absorbent core. In addition, if the through-holes are provided with a minimum fixed interval between them, the strength of the absorbent body is less likely to be reduced, and the excellent absorption property of the absorbent body (especially the diffusibility and absorption efficiency) can be stably exhibited.

According to the first embodiment of the invention, the core wrap 11' in the lengthwise groove sections 19 is joined to the bottom parts 20 of the lengthwise groove sections 19; however, in the absorbent article of the invention, the core wrap may be joined to the entirety of the lengthwise groove sections, i.e. to the bottom parts of the lengthwise groove sections and to the pair of side walls running in the lengthwise direction. This will help the lengthwise groove sections to retain their shapes even after the absorbent article has repeatedly absorbed excreted fluid (i.e., it will help maintain the function of the lengthwise groove sections as origins for folding), and therefore the flexibility can be maintained and as a result, the absorbent article will have an excellent feel during wear.

In the absorbent article of the invention, the lengthwise groove sections have widths that are preferably 0.5 to 3.0 times, more preferably 0.8 to 2.5 times and even more preferably 1.0 to 2.0 times the thickness of the absorbent body. If the lengthwise groove sections have widths of 0.5 to 3.0 times the thickness of the absorbent body, then even when the absorbent core has absorbed excreted fluid and expanded, it will not expand to the point of mutual contact between the pair of side walls that have swelled in the widthwise direction inside the lengthwise groove sections, and the lengthwise groove sections will be less likely to become plugged, and therefore fixed spaces extending in the lengthwise direction can be even more easily ensured in the lengthwise groove sections. As a result, the absorbent article will have flexibility and an excellent fitting property even after absorption of excreted fluid, and will be able to maintain the aforementioned excellent absorption property and rewetting property.

Throughout the present description, the thickness (mm) of the absorbent body is that measured in the following manner.

An FS-60DS by Daiei Kagaku Seiki Mfg. Co., Ltd. is prepared [measuring surface: 44 mm (diameter), measuring pressure: 3 g/cm²], five different locations of the absorbent body are pressed under standard conditions (temperature: 23±2°C, relative humidity: 50±5%), the thickness is measured after 10 seconds of pressing at each site, and the mean value of the five measured values is recorded as the thickness of the absorbent body.

According to the first embodiment of the invention, the compressed sections 23 are formed by compressing the two core wraps 11, 11', the diffusion sheet 13 and the absorbent core 9 inside the lengthwise groove sections; however, according to another embodiment of the invention, the compressed sections may be formed by compressing only the absorbent core inside the lengthwise groove sections. According to the invention it is not essential to provide a diffusion sheet in the absorbent article, and therefore when a diffusion sheet is not provided, the compressed sections may be formed by compressing the core wrap and the absorbent core inside the lengthwise groove sections. If the compressed sections of the absorbent article are formed by compressing the absorbent core and core wrap, then even when the absorbent core has absorbed excreted fluid and has expanded, the core wrap that is joined to the compressed sections of the bottom parts of the lengthwise groove sections will lie more reliably between the pair of side walls that have swelled in the widthwise direction, thus helping to more reliably ensure the fixed spaces running in the lengthwise direction inside the lengthwise groove sections.

Moreover, according to the first embodiment described above, the absorbent body 7 comprises the lengthwise groove sections 19 in all of the areas: the right area RA, the left area LA and the center area CA, in the widthwise direction W. If the absorbent article has such a construction, the lengthwise groove sections will be provided spanning the entire width in the widthwise direction of the absorbent body, and therefore excreted fluid can be absorbed from a wide region of the absorbent body, and a more excellent absorption property (especially absorption efficiency) and rewetting property as an absorbent article will be exhibited. Furthermore, since the lengthwise groove sections are formed in the one edge area and the other edge area in the widthwise direction of the absorbent core, an advantage is provided in that excreted fluid is less likely to leak from both edges in the widthwise direction of the absorbent article (i.e., in the area surrounding the legs of the wearer), via both edges in the widthwise direction of the absorbent body.

Furthermore, according to the first embodiment described above, the absorbent body 7 comprises the widthwise groove sections 21 in three areas: the front waist region RW, rear waist region LW and crotch region C, in the lengthwise direction L. If the absorbent article has such a construction, the absorbent article 1 will be able to exhibit an excellent fitting property in the lengthwise direction. Moreover, since excreted fluid that has flowed through the lengthwise groove sections 19 and diffused in the lengthwise direction L further flows into the widthwise groove sections 21 as well and diffuses in the widthwise direction W, being absorbed from a wide region of the absorbent body, the absorbent article 1 has an excellent rewetting property and as a result also has an excellent feel during wear.

Furthermore, according to the first embodiment, the core wrap 11' that is disposed on the clothing side surface of the absorbent core 9 is not joined to the bottom parts 22 of the widthwise groove sections 21 at the sections of the widthwise groove sections 21 that are not crossing with the lengthwise groove sections 19. Consequently, according to the first embodiment, although the widthwise groove sections 21 that have absorbed excreted fluid that has flowed in from the lengthwise groove sections 19 collapse by expansion of the absorbent core 9, the lengthwise groove sections 19 and the widthwise groove sections 21 where the excreted fluid has not reached (i.e., the widthwise groove sections 21 that have not absorbed excreted fluid) have not lost their function as excreted fluid diffusion channels (even when the lengthwise groove sections 19 have absorbed excreted fluid, they are able to ensure the fixed spaces 25 running in the lengthwise direction L as mentioned above, and therefore their function as excreted fluid diffusion channels is not lost), and during further subsequent excretions, the excreted fluid can flow through the lengthwise groove sections 19 (or the fixed spaces 25 running in the lengthwise direction L), and diffuse to the non-collapsed widthwise groove sections 21. Since this process is repeated when excreted fluid is supplied several times, the excreted fluid can be absorbed over a wide region of the absorbent body. Consequently, the absorbent article 1 can exhibit an even more excellent absorption property and rewetting property particularly for multiple excretions, and the absorbent article can be worn for prolonged periods.

According to the invention there is no limitation to this construction, and for example, according to another embodiment of the invention the core wrap may be joined to the bottom parts of the widthwise groove sections. If the absorbent article has such a construction, excreted fluid that has reached the clothing side surface of the absorbent body will readily flow through the widthwise groove sections and diffuse in the widthwise direction as well. In addition, similar to the lengthwise groove sections described above, the widthwise groove sections also easily retain their shapes even after the absorbent core has absorbed excreted fluid, and therefore the function of the widthwise groove sections as origins for folding and their function as excreted fluid diffusion channel can be adequately exhibited. As a result, even after absorption of excreted fluid (especially after repeated absorption of excreted fluid), the absorbent article easily deforms in the lengthwise direction as well along the body of the wearer, and has satisfactory flexibility in the lengthwise direction and widthwise direction, with an excellent fitting property, and can maintain the aforementioned excellent absorption property and rewetting property with an excellent feel during wear.

In addition, according to another embodiment of the invention, the core wrap may be joined to the bottom parts of the widthwise groove sections and the widthwise groove sections may include compressed sections formed by continuously or intermittently compressing portions of the widths of the widthwise groove sections along the widthwise groove sections. If the absorbent article has such a construction, the widthwise groove sections will have the same function as the lengthwise groove sections, and the absorbent article will exhibit an excellent fitting property and rewetting property and also an excellent feel during wear.

Furthermore, according to the first embodiment of the invention, the diffusion sheet 13 is disposed on the clothing side surface 17 of the core wrap 11' on the clothing side of the absorbent core 9, and is joined with the core wrap 11' at the sections that are joined to the bottom parts of the lengthwise groove sections 19. According to the first embodiment, therefore, even when the absorbent core 9 has absorbed excreted fluid and expanded, since the core wrap 11' and the diffusion sheet 13 lie between the pair of side walls 19b, 19c that have swelled in the widthwise direction W inside the lengthwise groove sections 19, it is possible to reliably ensure the aforementioned fixed spaces 25 running in the lengthwise direction L inside the lengthwise groove sections 19. As a result, the absorbent article 1 can more reliably exhibit the function of the lengthwise groove sections 19 as origins for folding and their function as excreted fluid diffusion channels.

In addition, since excreted fluid that has reached the lengthwise groove sections 19 is easily diffused in the lengthwise direction L of the absorbent body 7 by the lengthwise groove sections 19 and the diffusion sheet 13, it is possible to more rapidly cause diffusion of the excreted fluid in the lengthwise direction L of the absorbent body 7 while also allowing absorption over a wide region in the lengthwise direction L.

The absorbent article 1 therefore has flexibility and an excellent fitting property, excellent diffusibility in the lengthwise direction and an excellent rewetting property even after absorption of excreted fluid, and can also exhibit these effects more reliably.

According to the invention, the position where the diffusion sheet is disposed is not particularly restricted, and for example, the diffusion sheet may be disposed on the skin side surface of the core wrap on the skin side of the absorbent core, or the diffusion sheet may be disposed at a position between the core wrap and the absorbent core, which is on the skin side surface or the clothing side surface of the absorbent core. In particular, if the diffusion sheet is disposed at a position further on the skin side than the absorbent core, then excreted fluid supplied to the absorbent body will flow into the plurality of through-holes after having been diffused in the in-plane direction of the sheet by the diffusion sheet, and therefore the excreted fluid can be caused to migrate to the clothing side surface of the absorbent core through either a greater number of or more distant through-holes.

In addition, if the diffusion sheet is placed in a position that the diffusion sheet is not embedded in the lengthwise groove sections, then the diffusion sheet will be less likely to form a three-dimensional structure along the indentations of the lengthwise groove sections, and it will thus be possible to prevent increased rigidity (i.e., reduced flexibility) of the absorbent article.

Incidentally, since the diffusion sheet of the present invention is not an essential component, according to yet another embodiment of the invention the absorbent article may not include a diffusion sheet.

Furthermore, according to the first embodiment of the invention, the two core wraps 11, 11' and the diffusion sheet 13 each have holes communicating with the through-holes 24 of the lengthwise groove sections 19. Thus, excreted fluid supplied to the absorbent body will easily reach the clothing side surface of the absorbent body through the holes and through-holes, and the absorption property (especially the absorption rate) in the thickness direction of the absorbent body can be even further improved. According to the invention, there is no limitation to such a construction, and holes communicating with the through-holes of the lengthwise groove sections may instead be formed only in either the core wrap or the diffusion sheet.

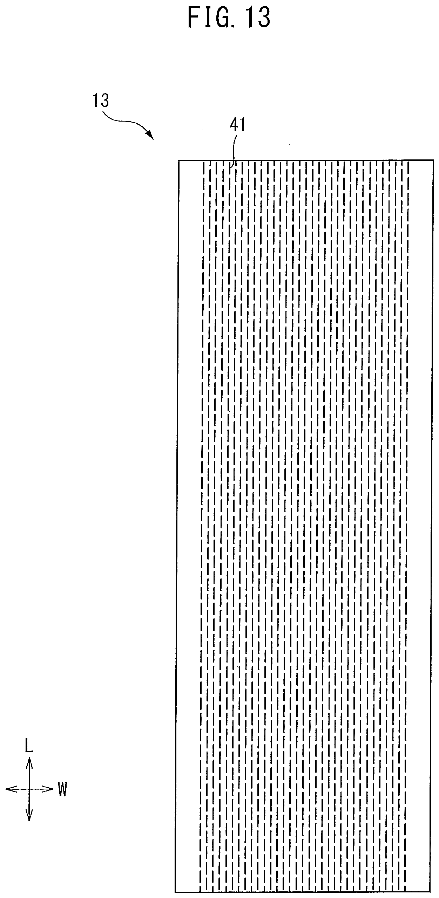

According to the invention, in order to lower the rigidity of the absorbent article, the diffusion sheet may have a plurality of slits running through the thickness direction of the diffusion sheet, as shown in Fig. 13. If the diffusion sheet has slits, when the diffusion sheet is joined to the bottom parts of the lengthwise groove sections and/or the widthwise groove sections, the slits will deform (open), reducing distortion of the absorbent body.

The slits are not particularly restricted, and may be straight linear slits (for example, slits 41 running in the lengthwise direction of the absorbent article as shown in Fig. 13, or slits running in the short direction of the absorbent article), or non-linear slits (for example, cross-shaped slits or curved slits. From the viewpoint of reducing distortion produced in the lengthwise groove sections, the slits are preferably slits running in the lengthwise direction of the absorbent article, and from the viewpoint of reducing strain produced in the widthwise groove sections, they are preferably slits running in the widthwise direction of the absorbent article.

In the absorbent article of the invention, the region including the absorbent body of the absorbent article has a flexural rigidity value of preferably 5.0 to 35.0 mN·m and more preferably 10.0 to 34.0 mN·m in the lengthwise direction and widthwise direction. If the flexural rigidity value is within this range, the absorbent article will deform more easily along the body of the wearer, and will have an excellent fitting property and thus an excellent feel during wear.

Throughout the present description, the flexural rigidity is that measured by the following procedure.
(1) An EX-0762 Torque Tester by Imada Co., Ltd. is prepared in a steady temperature and humidity room (20±2°C·60±5% RH). The load cell is set to a tension/compression of 50N, and the torque is set to IN.
(2) A sample is cut out, as shown in Table 1.
(3) At both edges in the direction of measurement of the sample, 50 mm × 20 mm size fabric tape is attached with the lengthwise direction perpendicular to the direction of measurement, in such a manner that the jig interval is as shown in Table 1, forming grip sections at both edges in the direction of measurement of the sample.
(4) The sample is mounted on the jig and the maximum torque value (mN·m) is measured under the conditions shown in Table 1.
(5) The measurement is carried out 5 times with different samples, and their mean value is used as the flexural rigidity value.

### [Table 1]

**Table 1**

| Measuring direction | Lengthwise direction | Widthwise direction |
|---|---|---|
| Sample (lengthwise direction/mm × widthwise direction/mm) | 120 × 60 | 60 × 120 |
| Tension /N | 1-2 | 1-2 |
| Jig interval /mm | 120 | 60 |
| Rotation speed /rpm | 50 | 50 |
| Rotation angle /° | 65 | 65 |
| Measuring angle /° | 60 | 60 |

In the absorbent article of the invention, both the absorbent core and the core wrap that are used may be ones commonly used in the technical field, without any particular restrictions. Examples for the absorbent core include pulp fiber and superabsorbent polymers, and examples for the core wrap include tissue.

In the absorbent article of the invention, the absorbent core has an average basis weight of preferably 100 to 400 g/m² and more preferably 200 to 300 g/m². In order to more advantageously exhibit the effect of the invention, both the lengthwise groove sections and the widthwise groove sections, as the low-basis-weight regions, have basis weights that are preferably no greater than 80%, more preferably no greater than 60% and even more preferably no greater than 40% of the average basis weight mentioned above. From the viewpoint of maintaining the structure as an absorbent body, both the lengthwise groove sections and widthwise groove sections as the low-basis-weight regions have basis weights of greater than 0 g/m², and preferably basis weights of at least 5%, more preferably at least 10% and even more preferably at least 20% of the average basis weight mentioned above.

For an embodiment in which the absorbent article includes a diffusion sheet, the diffusion sheet used may be one that is commonly used in the technical field, with no particular restrictions. For example, the diffusion sheet used may be the nonwoven fabric sheet described in Japanese Unexamined Patent Publication No. 2013-150799, which is a nonwoven fabric sheet comprising beaten pulp (a) and regenerated cellulose (b) in proportions of 20 to 40 parts by mass and 10 to 70 parts by mass, respectively, the beaten pulp (a) being fibrillated, with weight-weighted average fiber lengths in the range of 1.0 to 5.0 mm, and the density of the nonwoven fabric sheet being 50 to 280 mg/cm³.

Of these, the diffusion sheet is preferably a layered sheet comprising a first fiber layer including hydrophilic fibers with a mean fiber length of 25 mm to 64 mm, a second fiber layer including hydrophilic fibers with a mean fiber length of 25 mm to 64 mm, and a pulp-containing pulp fiber layer between these layers, more preferably the layered sheet is one formed by tangling the first fiber layer, the pulp fiber layer and the second fiber layer with a water stream, and even more preferably the layered sheet is one in which the first fiber layer, the pulp fiber layer and the second fiber layer are connected without using an adhesive.

Since a diffusion sheet having such a specific layered structure exhibits particularly excellent performance for causing diffusion of excreted fluid in the in-plane direction of the sheet, excreted fluid that has reached the diffusion sheet easily diffuses over a wider region in the lengthwise direction of the absorbent body, and the excreted fluid can be absorbed over a wider region of the absorbent body. Consequently, the absorbent article comprising such a diffusion sheet can exhibit an even more excellent absorption property and rewetting property as an absorbent article.

The "mean fiber length" referred to above is the mean fiber length measured according to "A7.1.1, Method A (Standard method) Method for measuring lengths of individual fibers on graduated glass plate", under "A7.1 Fiber length measurement" in appendix A of JIS L 1015:2010.

The absorbent core in the absorbent article of the invention, or in other words the absorbent core having low-basis-weight regions forming the lengthwise groove sections and widthwise groove sections, can be formed by the method described in Japanese Unexamined Patent Publication No. 2010-233839 or Japanese Unexamined Patent Publication No. 2014-136126, for example.

### Examples

The invention will now be explained in greater detail using examples and comparative examples, with the understanding that the invention is not limited only to these examples.

### Example 1

Pulp (basis weight: 200 g/m²) and a superabsorbent polymer (basis weight: 250 g/m²) were mixed and supplied to the molding recess of a suction drum to produce an absorbent core having a size of 350 mm × 120 mm (lengthwise direction × widthwise direction). The absorbent core had five lengthwise groove sections (groove width: 5 mm) disposed at 20 mm intervals on the second surface (clothing side surface), the lengthwise groove sections having a basis weight of 80 g/m².

The absorbent core was covered by two tissue sheets as core wraps (skin side tissue: basis weight of 16 g/m², 400 mm × 120 mm; second surface side (clothing side) tissue: basis weight of 16 g/m², 400 mm × 150 mm), with a hot-melt adhesive between them. Next, a diffusion sheet was attached to the second surface (clothing side surface) of the absorbent core covered with the two tissue sheets, via a hot-melt adhesive, and the absorbent core was pressed with a hydraulic press to adjust the thickness of the absorbent core, after which embossed sections (compressed sections) with widths of 3 mm and continuous in the lengthwise direction were formed to match the lengthwise groove sections, thereby integrating the absorbent core, the tissue and the diffusion sheet. In addition, eleven through-holes with diameters of 3 mm were formed at 10 mm intervals in each of the five lengthwise groove sections using a circular blade, to fabricate an absorbent body.

The diffusion sheet used was a layered sheet (basis weight: 50 g/m²) including a first fiber layer containing hydrophilic fibers with a mean fiber length of 25 mm to 64 mm, a second fiber layer containing hydrophilic fibers with a mean fiber length of 25 mm to 64 mm, and a pulp-containing pulp fiber layer between them.

A liquid-permeable sheet (air-through nonwoven fabric) as a top sheet was attached to the first surface (skin side surface) of the fabricated absorbent body, and a liquid-impermeable sheet (polyethylene film) as a back sheet was attached to the second surface (clothing side surface), to produce an absorbent article for Example 1.

### Example 2

An absorbent article for Example 2 was produced in the same manner as Example 1, except that five lengthwise groove sections (groove width: 5 mm) were arranged at 20 mm intervals and nine widthwise groove sections (lengthwise direction length (groove width): 5 mm) were arranged at 30 mm intervals on the second surface (clothing side surface) of the absorbent core, forming the lengthwise groove sections and widthwise groove sections in a lattice-like manner.

### Example 3

An absorbent article for Example 3 was produced in the same manner as Example 1, except that five lengthwise groove sections (groove width: 5 mm) were arranged at 20 mm intervals and nine intermittent widthwise groove sections (lengthwise direction length (groove width): 5 mm, widthwise direction length of each group of widthwise groove sections: 20 mm, widthwise direction interval of each group of widthwise groove sections: 5 mm) were arranged at 30 mm intervals, as shown in Fig. 12, on the second surface (clothing side surface) of the absorbent core.

### Comparative Example 1

An absorbent article for Comparative Example 1 was produced in the same manner as Example 1, except that no through-holes were formed in the lengthwise groove sections formed on the second surface (clothing side surface) of the absorbent core.

### Comparative Example 2

An absorbent article for Comparative Example 2 was produced in the same manner as Example 2, except that no through-holes were formed in the lengthwise groove sections formed on the second surface (clothing side surface) of the absorbent core.

### Comparative Example 3

An absorbent article for Comparative Example 3 was produced in the same manner as Example 3, except that no through-holes were formed in the lengthwise groove sections formed on the second surface (clothing side surface) of the absorbent core.

The produced absorbent articles of Examples 1 to 3 and Comparative Examples 1 to 3 were subjected to an absorption property test as specified below, and the absorption rate, rewetting amount and liquid-impermeable sheet side diffusion length were evaluated. The results are shown in Table 2.

### [Absorption property test]

(1) The absorbent article is set in a U-shaped appliance having an approximate U-shape as viewed from the side. The absorbent article is set in the U-shaped appliance so that positions of the center point in the lengthwise direction of the absorbent body and the center section (the lowest location) of the U-shaped appliance coincide.

### <First cycle>

(2) At the center point of the absorbent body, 80 mL of artificial urine (first time) is poured in from a burette at a speed of 80 mL/10 sec.
(3) The time from the start of pouring of the first artificial urine until the artificial urine in the U-shaped appliance disappears is recorded as the absorption time (80 mL).
(4) The outline (80 mL) of the artificial urine-diffused area of the liquid-impermeable sheet of the absorbent article is recorded 3 minutes after the start of pouring of the first artificial urine.

### <Second cycle>

(5) At 10 minutes from the start of pouring of the first artificial urine, 80 mL of artificial urine (second time) is poured in from a burette at a speed of 80 mL/10 sec, at the center point of the absorbent body.
(6) The time from the start of pouring of the second artificial urine until the artificial urine in the U-shaped appliance disappears is recorded as the absorption time (160 mL).
(7) The outline (160 mL) of the artificial urine-diffused area of the liquid-impermeable sheet of the absorbent article is recorded 3 minutes after the start of pouring of the second artificial urine.

### <Third cycle>

(8) The procedure of (5) to (7) is repeated, the absorption time (240 mL) is measured and the outline (240 mL) is recorded.

### <Fourth cycle>

(9) The procedure of (5) to (7) is repeated, the absorption time (320 mL) is measured and the outline (320 mL) is recorded.
(10) At 4 minutes after the start of pouring of the fourth artificial urine, the absorbent article is removed from the U-shaped appliance and the absorbent article is spread out onto an acrylic flat plate with the liquid-permeable sheet as the upper side, and allowed to stand for 1 minute.
(11) At 5 minutes from the start of pouring of the fourth artificial urine, 60 g of 100 mm × 100 mm filter paper is placed on the liquid-permeable sheet of the absorbent article with the artificial urine pouring point as the center. A 3.5 kg, 100 mm × 100 mm × 50 mm (height) deadweight is then placed over it. The prior weight of the filter paper before the test is measured.
(12) At 8 minutes from the start of pouring of the fourth artificial urine, the deadweight is removed, the weight of the filter paper is measured, the weight of the filter paper before the test is subtracted and the difference is recorded as the rewetting amount.
(13) The diffusion length of the artificial urine in the lengthwise direction on the liquid-impermeable sheet side (back sheet side) of the absorbent article is measured from the outline of the artificial urine-diffused area (160 mL to 320 mL).

The artificial urine was prepared by dissolving 200 g of urea, 80 g of sodium chloride, 8 g of magnesium sulfate, 3 g of calcium chloride and approximately 1 g of dye: Blue #1 in 10 L of ion-exchanged water.

### [Table 2]

**Table 2**

| | | | Example 1 | Comp. Example 1 | Example 2 | Comp. Example 2 | Example 3 | Comp. Example 3 |
|---|---|---|---|---|---|---|---|---|
| Absorbent body | Lengthwise groove sections | | yes | yes | yes | yes | yes | yes |
| | Embossed sections in lengthwise groove sections | | yes | yes | yes | yes | yes | yes |
| | Through-holes in lengthwise groove sections | | yes | no | yes | no | yes | no |
| | Widthwise groove sections | | no | no | yes (lattice) | yes (lattice) | yes (intermittent) | yes (intermittent) |
| | Absorbent body thickness /mm | | 3.5 | 3.3 | 3.6 | 3.4 | 3.9 | 3.7 |
| Diffusion sheet | | | yes | yes | yes | yes | yes | yes |
| Absorption property test | Absorption time /sec | 80 mL | 15 | 14 | 14 | 15 | 14 | 16 |
| | | 160 mL | 12 | 13 | 11 | 14 | 14 | 15 |
| | | 240 mL | 17 | 18 | 17 | 19 | 21 | 19 |
| | | 320 mL | 26 | 33 | 26 | 29 | 28 | 27 |
| | Rewetting amount /g | 320 mL | 42 | 56 | 50 | 49 | 41 | 36 |
| | Diffusion length /mm (back sheet side) | 80 mL | 216 | 204 | 205 | 209 | 215 | 215 |
| | | 160 mL | 227 | 241 | 226 | 225 | 226 | 223 |
| | | 240 mL | 268 | 298 | 298 | 276 | 277 | 267 |
| | | 320 mL 320 | 312 | 348 | 314 | 334 | 332 | 320 |

It was found that, although each of the absorbent articles of Examples 1 to 3 had approximately the same diffusion length in the lengthwise direction on the liquid-impermeable sheet side (back sheet side), as the absorbent articles of Comparative Examples 1 to 3 that did not have through-holes formed in the lengthwise groove sections, their absorption rates were relatively faster. Moreover, the absorbent articles of Examples 1 to 3 were easily folded and had satisfactory flexibility, not only before absorption but also after absorption of the artificial urine.

### Reference Sign List

1 Absorbent article
3 Liquid-permeable sheet
5 Liquid-impermeable sheet
7 Absorbent body
9 Absorbent core
11, 11' Core wrap
13 Diffusion sheet
15 Skin side surface
17 Clothing side surface
19 Lengthwise groove section
19a Low-basis-weight region
19b Side wall
19c Side wall
20 Bottom part
21 Widthwise groove section
21a Low-basis-weight region
21b Partitioned widthwise groove section
21c Widthwise edge
22 Bottom part
23 Compressed section
24 Through-hole
L Lengthwise direction
W Widthwise direction
T Thickness direction

## Claims

1. An absorbent article (1) having a lengthwise direction and a widthwise direction and including a liquid-permeable sheet (3), a liquid-impermeable sheet (5) and an absorbent body (7) between the liquid-permeable sheet and liquid-impermeable sheet, wherein:
the absorbent body (7) comprises an absorbent core (9) having a first surface on a side facing the liquid-permeable sheet, and a second surface on a side facing the liquid-impermeable sheet (5), and a core wrap (11) covering the absorbent core,
the absorbent core (9) comprising a lengthwise groove section (19) that is a groove section indented from the second surface and extending in the lengthwise direction, and with a bottom part, and having lower basis weight than an average basis weight of the absorbent core (9), and a widthwise groove section (21) that is a groove section indented from the second surface and extending in the widthwise direction, and having lower basis weight than the average basis weight of the absorbent core (9),
the lengthwise groove section (19) including a plurality of through-holes running from the first surface to the second surface,
the widthwise groove section (21) being provided intermittently in the widthwise direction on the second surface of the absorbent core (9), and
the core wrap (11) being joined to the bottom part of the lengthwise groove section (19).

2. The absorbent article according to claim 1, wherein the lengthwise groove section (19) has a width of 0.5 to 3.0 times a thickness of the absorbent body.

3. The absorbent article according to claim 1 or 2, wherein the lengthwise groove section (19) comprises a compressed section where a portion of the bottom part is compressed in the widthwise direction, the compressed section running continuously or intermittently along the lengthwise groove section (19).

4. The absorbent article according to claim 3, wherein the compressed section is formed by compressing the absorbent core (9) and core wrap (11).

5. The absorbent article according to any one of claims 1 to 4, wherein openings of the through-holes on the first surface of the absorbent core (9) have a maximum inner diameter that is 0.2 to 0.8 times a width of the lengthwise groove section (19).

6. The absorbent article according to any one of claims 1 to 5, wherein the plurality of through-holes are arranged in the lengthwise groove section (19) at intervals that are longer than the maximum inner diameter of the openings on the first surface of the absorbent core (9).

7. The absorbent article according to any one of claims 1 to 6, wherein the absorbent body (7) is partitioned into one edge area, another edge area and a center area between them, by imaginary lines that divide the absorbent body (9) into three equal portions in the widthwise direction, and the absorbent core (9) comprises the lengthwise groove section (19) in all of the areas including the one edge area, the other edge area and the center area.

8. The absorbent article according to any one of claims 1 to 7, wherein the absorbent article (1) is partitioned into three regions in the lengthwise direction: a front waist region, a rear waist region and a crotch region between them, the absorbent body (7) being disposed across the three regions and the absorbent core (9) comprising the widthwise groove section (21) in all of the three regions.

9. The absorbent article according to any one of claims 1 to 7 or 8, wherein the core wrap (11) is not joined to the bottom part of the widthwise groove section (21) at a section of the widthwise groove section (21) that is not crossing with the lengthwise groove section (19).

10. The absorbent article according to any one of claims 1 to 7 or 9, which comprises a diffusion sheet (13) between the core wrap disposed on the second surface side of the absorbent core (9) and the liquid-impermeable sheet (5), the diffusion sheet being joined with the core wrap disposed on the second surface side of the absorbent core (9), at a section that is joined to the bottom part of the lengthwise groove section (19).

11. The absorbent article according to claim 10, wherein the diffusion sheet (13) is a layered sheet including a first fiber layer containing hydrophilic fibers with a mean fiber length of 25 mm to 64 mm, a second fiber layer containing hydrophilic fibers with a mean fiber length of 25 mm to 64 mm, and a pulp-containing pulp fiber layer between them.

12. The absorbent article according to claim 10 or 11, wherein the core wrap (11) and the diffusion sheet (13) each have holes that communicate with the through-holes of the lengthwise groove section (19).

13. The absorbent article according to any one of claims 10 to 12, wherein the diffusion sheet (13) has a plurality of slits running through the thickness direction.

## Patentansprüche

1. Absorbierender Artikel (1) mit einer Längsrichtung und einer Breitenrichtung und der eine flüssigkeitsdurchlässige Lage (3), eine flüssigkeitsundurchlässige Lage (5) und einen absorbierenden Körper (7) zwischen der flüssigkeitsdurchlässigen Lage und der flüssigkeitsundurchlässigen Lage umfasst, wobei:
der absorbierende Körper (7) einen absorbierenden Kern (9) mit einer ersten Oberfläche auf einer Seite, die der flüssigkeitsdurchlässigen Lage zugewandt ist, und einer zweiten Oberfläche auf einer Seite, die der flüssigkeitsundurchlässigen Lage (5) zugewandt ist, und eine Kernwicklung (11), die den absorbierenden Kern bedeckt, umfasst,
der absorbierende Kern (9) einen Längsnutabschnitt (19), der ein von der zweiten Oberfläche eingelassener und sich in Längsrichtung erstreckender Nutabschnitt ist, und mit einem Unterteil, der ein geringeres Flächengewicht als ein durchschnittliches Flächengewicht des absorbierenden Kerns (9) aufweist, und einen Breitennutabschnitt (21), der ein von der zweiten Oberfläche eingelassener und sich in Längsrichtung erstreckender Nutabschnitt ist, und ein geringeres Flächengewicht als das durchschnittliche Flächengewicht des absorbierenden Kerns (9) aufweist, umfasst,
der Längsnutabschnitt (19) eine Vielzahl von Durchgangsbohrungen umfasst, die von der ersten Oberfläche zur zweiten Oberfläche verlaufen,
der Breitennutabschnitt (21), der intermittierend in Breitenrichtung auf der zweiten Oberfläche des Absorptionskerns (9) vorgesehen ist, und
die Kernwicklung (11) mit dem unteren Teil des Längsnutabschnitts (19) verbunden ist.

2. Absorbierender Artikel gemäß Anspruch 1, wobei der Längsnutabschnitt (19) eine Breite von 0,5 bis 3,0 mal einer Dicke des absorbierenden Körpers aufweist.

3. Absorbierender Artikel gemäß Anspruch 1 oder 2, wobei der Längsnutabschnitt (19) einen komprimierten Abschnitt umfasst, in dem ein Teil des Unterteils in Breitenrichtung komprimiert ist, wobei der komprimierte Abschnitt kontinuierlich oder intermittierend entlang des Längsnutabschnitts (19) verläuft.

4. Absorbierender Artikel gemäß Anspruch 3, wobei der komprimierte Abschnitt durch Zusammendrücken des absorbierenden Kerns (9) und der Kernwicklung (11) ausgebildet ist.

5. Absorbierender Artikel gemäß einem der Ansprüche 1 bis 4, wobei Öffnungen der Durchgangsbohrungen auf der ersten Oberfläche des absorbierenden Kerns (9) einen maximalen Innendurchmesser aufweisen, der das 0,2- bis 0,8-fache der Breite des Längsnutabschnitts (19) beträgt.

6. Absorbierender Artikel gemäß einem der Ansprüche 1 bis 5, wobei die Vielzahl von Durchgangsbohrungen im Längsnutabschnitt (19) in Abständen angeordnet ist, die länger sind als der maximale Innendurchmesser der Öffnungen auf der ersten Oberfläche des absorbierenden Kerns (9).

7. Absorbierender Artikel gemäß einem der Ansprüche 1 bis 6, wobei der absorbierende Körper (7) in einen Randbereich, einen weiteren Randbereich und einen mittleren Bereich dazwischen durch imaginäre Linien unterteilt ist, die den absorbierenden Körper (9) in drei gleiche Teile in Breitenrichtung teilen, und der absorbierende Kern (9) den Längsnutabschnitt (19) in allen Bereichen einschließlich des einen Randbereichs, des weiteren Randbereichs und der mittleren Bereichs umfasst.

8. Absorbierender Artikel gemäß einem der Ansprüche 1 bis 7, wobei der absorbierende Artikel (1) in Längsrichtung in drei Bereiche unterteilt ist: einen vorderen Taillenbereich, einen hinteren Taillenbereich und einen Schrittbereich dazwischen, wobei der absorbierende Körper (7) über die drei Bereiche angeordnet ist und der absorbierende Kern (9) den breiten Nutabschnitt (21) in allen der drei Bereichen umfasst.

9. Absorbierender Artikel gemäß einem der Ansprüche 1 bis 7 oder 8, wobei die Kernwicklung (11) nicht mit dem unteren Teil des Breitennutabschnitts (21) an einem Abschnitt des Breitennutabschnitts (21) verbunden ist, der sich nicht mit dem Längsnutabschnitt (19) überschneidet.

10. Absorbierender Artikel gemäß einem der Ansprüche 1 bis 7 oder 9, der eine Diffusionslage (13) zwischen der auf der zweiten Oberflächenseite des absorbierenden Kerns (9) angeordneten Kernwicklung und der flüssigkeitsundurchlässigen Lage (5) umfasst, wobei die Diffusionslage mit der auf der zweiten Oberflächenseite des absorbierenden Kerns (9) angeordneten Kernwicklung an einem Abschnitt verbunden ist, der mit dem unteren Teil des Längsnutabschnitts (19) verbunden ist.

11. Absorbierender Artikel gemäß Anspruch 10, wobei die Diffusionslage (13) eine geschichtete Lage ist, die eine erste Faserschicht mit hydrophilen Fasern mit einer mittleren Faserlänge von 25 mm bis 64 mm, eine zweite Faserschicht mit hydrophilen Fasern mit einer mittleren Faserlänge von 25 mm bis 64 mm und eine pulpenhaltige Pulpfaserschicht zwischen ihnen umfasst.

12. Absorbierender Artikel gemäß Anspruch 10 oder 11, wobei die Kernwicklung (11) und die Diffusionslage (13) jeweils Löcher aufweisen, die mit den Durchgangslöchern des Längsnutabschnitts (19) verbunden sind.

13. Absorbierender Artikel gemäß einem der Ansprüche 10 bis 12, wobei die Diffusionslage (13) eine Vielzahl von Schlitzen aufweist, die durch die Dickenrichtung verlaufen.

## Revendications

1. Article absorbant (1) ayant une direction dans le sens de la longueur et une direction dans le sens de la largeur et incluant une feuille perméable aux liquides (3), une feuille imperméable aux liquides (5) et un corps absorbant (7) entre la feuille perméable aux liquides et la feuille imperméable aux liquides, dans lequel :
le corps absorbant (7) comprend un noyau absorbant (9) ayant une première surface sur un côté tourné vers la feuille perméable aux liquides, et une seconde surface sur un côté tourné vers la feuille imperméable aux liquides (5), et une enveloppe de noyau (11) couvrant le noyau absorbant,
le noyau absorbant (9) comprenant une section rainure dans le sens de la longueur (19) qui est une section rainure creusée depuis la seconde surface et s'étendant dans la direction dans le sens de la longueur, et avec une partie de fond,
et ayant un poids de base inférieur à un poids de base moyen du noyau absorbant (9), et une section rainure dans le sens de la largeur (21) qui est une section rainure creusée depuis la seconde surface et s'étendant dans la direction dans le sens de la largeur, et ayant un poids de base inférieur au poids de base moyen du noyau absorbant (9),
la section rainure dans le sens de la longueur (19) incluant une pluralité de trous traversants s'étalant de la première surface à la seconde surface,
la section rainure dans le sens de la largeur (21) étant disposée par intermittence dans la direction dans le sens de la largeur sur la seconde surface du noyau absorbant (9), et
l'enveloppe de noyau (11) étant reliée à la partie de fond de la section rainure dans le sens de la longueur (19).

2. Article absorbant selon la revendication 1, dans lequel la section rainure dans le sens de la longueur (19) a une largeur de 0,5 à 3,0 fois une épaisseur du corps absorbant.

3. Article absorbant selon la revendication 1 ou 2, dans lequel la section rainure dans le sens de la longueur (19) comprime une section comprimée où une portion de la partie de fond est comprimée dans la direction dans le sens de la largeur, la section comprimée s'étalant en continu ou par intermittence le long de [a section rainure dans le sens de la longueur (19).

4. Article absorbant selon la revendication 3, dans lequel la section comprimée est formée en comprimant les noyau absorbant (9) et enveloppe de noyau (11).

5. Article absorbant selon l'une quelconque des revendications 1 à 4, dans lequel des ouvertures des trous traversants sur la première surface du noyau absorbant (9) ont un diamètre intérieur maximum qui fait 0,2 à 0,8 fois une largeur de la section rainure dans le sens de la longueur (19).

6. Article absorbant selon l'une quelconque des revendications 1 à 5, dans lequel la pluralité de trous traversants sont agencés dans la section rainure dans le sens de la longueur (19) à intervalles qui sont plus longs que le diamètre intérieur maximum des ouvertures sur la première surface du noyau absorbant (9),

7. Article absorbant selon l'une quelconque des revendications 1 à 6, dans lequel le corps absorbant (7) est séparé en une zone de bord, en une autre zone de bord et en une zone centrale entre elles, par des lignes imaginaires qui divisent le corps absorbant (9) en trois portions égales dans la direction dans le sens de la largeur, et le noyau absorbant (9) comprend la section rainure dans le sens de la longueur (19) dans toutes les zones incluant la une zone de bord, l'autre zone de bord et la zone centrale,

8. Article absorbant selon l'une quelconque des revendications 1 à 7, dans lequel l'article absorbant (1) est séparé en trois régions dans la direction dans le sens de la longueur : une région taille avant, une région taille arrière et une région entrejambe entre elles, le corps absorbant (7) étant disposé à travers les trois régions et le noyau absorbant (9) comprenant la section rainure dans le sens de la largeur (21) dans la totalité des trois régions.

9. Article absorbant selon l'une quelconque des revendications 1 à 7 ou 8, dans lequel l'enveloppe de noyau (11) n'est pas reliée à la partie de fond de la section rainure dans le sens de la largeur (21) au niveau d'une section de la section rainure dans le sens de la largeur (21) qui ne coupe pas la section rainure dans le sens de la longueur (19).

10. Article absorbant selon l'une quelconque des revendications 1 à 7 ou 9, qui comprend une feuille de diffusion (13) entre l'enveloppe de noyau disposée sur le côté seconde surface du noyau absorbant (9) et la feuille imperméable aux liquides (5), la feuille de diffusion étant reliée à l'enveloppe de noyau disposée sur le côté seconde surface du noyau absorbant (9), au niveau d'une section qui est reliée à la partie de fond de la section rainure dans le sens de la longueur (19).

11. Article absorbant selon la revendication 10, dans lequel la feuille de diffusion (13) est une feuille en couches incluant une première couche de fibres contenant des fibres hydrophiles ayant une longueur de fibre moyenne de 25 mm à 64 mm, une deuxième couche de fibres contenant des fibres hydrophiles ayant une longueur de fibre moyenne de 25 mm à 64 mm, et une couche de fibres de pâte à papier contenant de la pâte à papier entre elles.

12. Article absorbant selon la revendication 10 ou 11, dans lequel l'enveloppe de noyau (11) et la feuille de diffusion (13) comportent chacune des trous qui communiquent avec les trous traversants de la section rainure dans le sens de la longueur (19).

13. Article absorbant selon l'une quelconque des revendications 10 à 12, dans lequel la feuille de diffusion (13) comporte une pluralité de fentes s'étalant dans la direction d'épaisseur.
